# EUROPÄISCHE PATENTSCHRIFT

(11) **EP 3 784 327 B1**
(45) Veröffentlichungstag und Bekanntmachung des Hinweises auf die Patenterteilung: **27.03.2024**
(21) Anmeldenummer: 19718633.1
(22) Anmeldetag: 11.04.2019
(51) Int. Cl.: A61N 1/05, A61N 1/36

(54) **ELEKTRODENKÖRPER EINER ELEKTRODENANORDNUNG UND ELEKTRODENANORDNUNG ZUR ELEKTRISCHEN STIMULATION SOWIE VERFAHREN ZUR HERSTELLUNG EINER ELEKTRODENANORDNUNG**
ELECTRODE BODY OF AN ELECTRODE ASSEMBLY AND ELECTRODE ASSEMBLY FOR ELECTRICAL STIMULATION, AND METHOD FOR PRODUCING AN ELECTRODE ASSEMBLY
CORPS D'ÉLECTRODE D'UN ENSEMBLE D'ÉLECTRODES ET ENSEMBLE D'ÉLECTRODES DE STIMULATION ÉLECTRIQUE ET PROCÉDÉ DE FABRICATION D'UN ENSEMBLE D'ÉLECTRODES

(30) Priorität: 27.04.2018 DE 102018110239
(43) Veröffentlichungstag der Anmeldung: 03.03.2021
(73) Patentinhaber: PRECISIS GmbH, 69117 Heidelberg (DE)
(72) Erfinder: TITTELBACH, Michael, 69126 Heidelberg (DE); LIEDLER, Angela, 82319 Starnberg (DE); REMMERT, Gregor, 69115 Heidelberg (DE); JAGSCHIES, Lasse, 69118 Heidelberg (DE); GÖTTSCHE, Thorsten, 79618 Rheinfelden (DE); GRÄFE, Maik, 79618 Rheinfelden (DE)
(74) Vertreter: Günther, Constantin
(86) Internationale Anmeldenummer: PCT/EP2019/059278
(87) Internationale Veröffentlichungsnummer: WO 2019/206664

(56) Entgegenhaltungen:
- EP-B1- 2 038 004
- EP-B1- 2 038 004
- WO-A1-2018/072894
- WO-A2-2005/002665
- US-A1- 2015 148 864

## Beschreibung

Die Erfindung betrifft einen Elektrodenkörper einer Elektrodenanordnung zur elektrischen Stimulation von Gewebe eines Lebewesens, insbesondere für die Neurostimulation. Die Erfindung betrifft weiterhin eine Elektrodenanordnung zur elektrischen Stimulation von Gewebe eines Lebewesens, insbesondere für die Neurostimulation.

Derartige Elektrodenkörper und Elektrodenanordnungen erzeugen ein elektrisches Feld im Bereich von Gewebe eines Lebewesens zur elektrischen Stimulation dieses Gewebes, wobei die Elektroden durch eine Energiequelle gespeist werden. Die Begriffe Elektrode und Elektrodenkörper sind im Wesentlichen gleichzusetzen, wobei der Begriff Elektrodenkörper nachfolgend insbesondere zur Beschreibung des Aufbaus und der Struktur solcher Elektroden einer Elektrodenanordnung verwendet wird.

EP 2 709 716 A1 zeigt eine Vorrichtung zur Implantation in oder an einem Kopf, wobei die Vorrichtung ein Gehäuse hat, in dem mehrere Elektroden angeordnet sind, wobei die Elektroden ein elektrisches Feld in einer bestimmten Region des Kopfes erzeugen. Die Vorrichtung ist dabei zum Empfangen und Senden von Daten durch W-LAN eingerichtet.

WO 2005/002665 A2 offenbart ein Elektrodensystem zur Implantation in einem Menschen, mit zwei Elektrodenreihen, wobei in einer Elektrodenreihe mehrere Elektroden auf einer Linie nebeneinander angeordnet sind. Die Elektrodenreihen können mit unterschiedlichen Spannungen angesteuert werden, wobei ein elektrisches Feld erzeugt wird.

EP 2 038 004 B1 zeigt ein Elektrodensystem zur Implantierung am Kopf außerhalb des Schädels, mit mehreren Scheibenelektroden, wobei die Scheibenelektroden in einer Matrix angeordnet sind. Dabei können ausgewählte Scheibenelektroden je nach Anwendung durch ein Umschalt-Subsystem angesteuert werden, um ein gewünschtes elektrisches Feld zu erzeugen, ohne die Position der Scheibenelektroden verändern zu müssen.

Ausgehend hiervon ist es Aufgabe der vorliegenden Erfindung, einen verbesserten Elektrodenkörper einer Elektrodenanordnung und eine verbesserte Elektrodenanordnung zu schaffen.

Die Aufgabe wird durch den Elektrodenkörper mit den Merkmalen des Anspruchs 1 gelöst. Vorteilhafte Ausführungsformen sind in den Unteransprüchen beschrieben.

Dementsprechend ist ein Elektrodenkörper einer Elektrodenanordnung zur elektrischen Stimulation von Gewebe eines Lebewesens, insbesondere für die Neurostimulation vorgesehen. Hierbei ist der Elektrodenkörper dazu eingerichtet, an einer Stelle zwischen dem Schädel und der Kopfschwarte eines Lebewesens angeordnet zu werden. Der Elektrodenkörper weist eine Stimulationsoberfläche auf, die dazu eingerichtet ist, in Kontakt mit dem Gewebe des Lebewesens gebracht zu werden, um eine elektrische Stimulation des Gewebes durch Wechselstrom- und/oder Gleichstrom-Pulse zu erzeugen. Hiervon sind auch kombinierte oder überlagerte Wechsel- und Gleichstrompulse umfasst, beispielsweise auch asymmetrische Pulse, die nicht den gleichen momentanen oder integralen Stromfluss in positiver und negativer Richtung aufweisen.

Der Elektrodenkörper kann eine Stimulationsoberfläche mit einem Flächeninhalt von wenigstens 50 mm² haben. Das von dem Elektrodenkörper einer Elektrodenanordnung erzeugte elektrische Feld wird von dem Schädel des Lebewesens abgeschwächt. Indem durch eine wenigstens 50 mm² große Stimulationsoberfläche des Elektrodenkörpers ein ausreichend starkes elektrisches Feld erzeugt wird, ohne die Stimulationsoberfläche durch elektrochemische Prozesse zu beschädigen, kann diese Abschwächung durch den Schädel besser kompensiert werden. Des Weiteren erweist sich die mindestens 50 mm² große Stimulationsoberfläche als vorteilhaft, wenn elektrische Pulse mit einer langen Pulsdauer verwendet werden, da sie bei einer konstanten Stromstärke zu geringeren Stromdichten an der Stimulationsoberfläche führen und somit irreversible elektrochemische Prozesse verringert werden. Überdies wird die potentiell stimulierbare Hirnfläche vergrößert. Die vergleichsweise große Stimulationsoberfläche wirkt sich auch günstig auf das Signal-zu-Rausch-Verhältnis aus.

Alternativ oder zusätzlich kann der Elektrodenkörper eine Stimulationsoberfläche mit einem Flächeninhalt von wenigstens 20 mm² und zusätzlich eine die wirksame Stimulationsoberfläche vergrößernde Oberflächenbehandlung aufweisen. Beispielsweise kann bei einer solchen Oberflächenbehandlung eine rauere oder genoppte Oberfläche oder auch eine fraktale Oberfläche erzeugt werden, wodurch sich eine Oberflächenvergrößerung ergibt und die Übergangsimpedanz zwischen Elektrodenkörper und Gewebe verringert wird, sodass das Signal-zu-Rausch-Verhältnis verbessert wird.

Alternativ oder zusätzlich kann der Elektrodenkörper eine Fixierungsstruktur für die Fixierung des Elektrodenkörpers am Gewebe eines Lebewesens haben.

Alternativ oder zusätzlich kann der Elektrodenkörper einen Leiteranschluss haben, über den der Elektrodenkörper durch Löten, Kleben, Schweißen oder eine anderweitige Verbindungstechnik mit einem elektrischen Leiter zur elektrischen Verbindung des Elektrodenkörpers mit einem elektrischen Gerät oder einem anderen Elektrodenkörper verbunden werden kann.

Alternativ oder zusätzlich kann der Elektrodenkörper bis auf die Stimulationsoberfläche von einem elektrisch isolierenden Trägermaterial vollständig umgeben sein. Dies ermöglicht die einfache Anordnung und Fixierung des Elektrodenkörpers in einer übergeordneten Struktur, insbesondere einer Elektrodenanordnung. Zudem wird durch das elektrisch isolierende Trägermaterial das Auftreten von Leck- und Kurzschlussströmen verhindert oder zumindest reduziert. Hierbei kann der Elektrodenkörper auch von mehreren elektrisch isolierenden Trägermaterialien umgeben sein. Beispielsweise kann auf den Elektrodenkörper ein Polyimid-Substrat aufgebracht sein, das zusätzlich in Silikon eingebettet ist.

Erfindungsgemäß hat der Elektrodenkörper über den Umfang verteilt angeordnete Ausnehmungen zur Fixierung des Elektrodenkörpers in einem Trägermaterial. Derartige Ausnehmungen haben den Vorteil, dass der Elektrodenkörper besser in dem Trägermaterial fixiert gehalten werden kann, indem eine verbesserte formschlüssige Verbindung erreicht werden kann. Bei der Herstellung gelangt das zunächst flüssige Trägermaterial wie zum Beispiel ein Silikon in die Ausnehmungen des Elektrodenkörpers, wobei nach Aushärten des Trägermaterials der Elektrodenkörper fest mit dem Trägermaterial verbunden ist. Die Ausnehmungen können zum Beispiel ringförmig um eine Befestigungsöffnung des Elektrodenkörpers und/oder entlang des Elektrodenrands angeordnet sein.

In einer besonders günstigen Ausgestaltung hat das Trägermaterial eine erste zumindest teilweise umlaufende Dichtlippe an einer freiliegenden Kontaktierungsseite des Elektrodenkörpers. Eine umlaufende Dichtlippe hat den Vorteil, dass der Elektrodenkörper vor unerwünschten Wechselwirkungen an der implantierten Stelle mit der Umgebung geschützt wird, wobei die umlaufende Dichtlippe eine Isolierung bereitstellt, die auch bei Deformation des Elektrodenkörpers oder einer den Elektrodenkörper aufnehmenden Elektrodenanordnung intakt bleibt. Im Gegensatz zu aus dem Stand der Technik bekannten Elektrodenkörpern und Elektrodenanordnungen können so die Haltbarkeit verbessert und Kurzschlüsse durch Elektrolyte oder Gewebe, das sich zwischen Elektrode und Schädel befindet, verhindert oder zumindest deutlich reduziert werden. Weiterhin wird das Signal-zu-Rausch-Verhältnis verbessert. Es ist denkbar, dass die erste umlaufende Dichtlippe in einem Stück aus dem Trägermaterial geformt sein kann.

Vorteilhaft ist es, wenn die erste zumindest teilweise umlaufende Dichtlippe den Elektrodenkörper um 0,05 mm bis 1 mm, insbesondere 0,1 mm bis 0,3 mm, überragt. Es hat sich gezeigt, dass diese Abmessungen die Flexibilität des Elektrodenkörpers und einer den Elektrodenkörper aufnehmenden Elektrodenanordnung gewährleisten und gleichzeitig eine effiziente Isolierung des Elektrodenkörpers vor äußeren Einflüssen bereitstellen. Die Dichtlippe kann den Elektrodenkörper dabei in jeder Raumrichtung überragen. Beispielsweise kann die Dichtlippe den Elektrodenkörper in Normalenrichtung der Stimulationsoberfläche und/oder zentripetal zu der Stimulationsoberfläche überragen.

Gemäß einer vorteilhaften Ausführungsform ist der Elektrodenkörper kreisförmig, ellipsenförmig, ringförmig oder bohnenförmig ausgebildet. Mit einer solchen Form ergibt sich eine gute Anlage des Elektrodenkörpers an das Gewebe des Lebewesens.

Günstig ist es, wenn der Leiteranschluss des Elektrodenkörpers als ein tangentialer Leiteranschluss ausgebildet ist. Ein tangentialer Leiteranschluss verläuft dabei orthogonal zur Verbindungslinie zwischen Berührungspunkt und Mittelpunkt des Elektrodenkörpers. Ein über den Leiteranschluss an den Elektrodenkörper anschließbarer Leiter berührt den Elektrodenkörper wie eine Tangente. Dies hat den Vorteil, dass die Verbindungsstelle bei mechanischer Belastung des elektrischen Leiters wie zum Beispiel durch eine Zugkraft entlastet wird, sodass die Wahrscheinlichkeit einer Beschädigung der Verbindungsstelle und damit ein Ausfall der Elektrode verringert wird. Der elektrische Leiter kann dabei zum Beispiel an den Elektrodenkörper angeschweißt oder aufgelötet werden.

Es ist jedoch auch denkbar, dass der Leiteranschluss des Elektrodenkörpers als ein rechtwinkliger Leiteranschluss ausgebildet ist. Unter einem rechtwinkligen Leiteranschluss wird ein Leiteranschluss verstanden, der die Kontur- oder Umfangslinie des Elektrodenkörpers im Anschlussbereich senkrecht schneidet, sodass sich ein rechter Winkel zwischen dem Leiter und der Kontur- oder Umfangslinie im Anschlussbereich ausbildet. Im Vergleich zum tangentialen Leiteranschluss verläuft der rechtwinklige Leiteranschluss parallel statt orthogonal zur Verbindungslinie zwischen Berührungspunkt und Mittelpunkt des Elektrodenkörpers. Diese Leiteranschlussform ist beispielsweise bei Polyimid-Substraten vorteilhaft.

In einer vorteilhaften Ausführungsform ist am Leiteranschluss ein elektrischer Leiter zur elektrischen Verbindung des Elektrodenkörpers mit einem elektrischen Gerät oder einem anderen Elektrodenkörper angeschlossen.

Vorteilhaft ist es, wenn der elektrische Leiter mäanderförmig verläuft. Eine mäanderförmige Anordnung verringert die Belastung des elektrischen Leiters, insbesondere in dessen Anschlussbereich, die zum Beispiel durch Biegen und Ziehen einer den Elektrodenkörper aufnehmenden Elektrodenanordnung entstehen können. Durch die Anordnung des Leiters auf einer gebogenen Bahn wird die Entlastung weiterhin verbessert. Vorteilhafterweise verläuft die gebogene Bahn über einen Winkelbereich von 40° bis 100°, ausgehend von einem 360°-System, um die Primärelektrode.

Gemäß einer günstigen Ausführungsform ist die Verbindungsstelle des elektrischen Leiters mit dem Elektrodenkörper abgedichtet, insbesondere mit einem Epoxidharz abgedichtet. Dies hat den Vorteil, dass die empfindliche Verbindungsstelle vor äußeren Einflüssen wie zum Beispiel Flüssigkeiten geschützt wird, die zu einem Ausfall, zum Beispiel durch Korrosion, der Elektrode führen würde.

In einer vorteilhaften Ausführungsform besteht der elektrische Leiter aus einem Material oder einer Materialzusammensetzung, die in ionenhaltiger Lösung kein elektrochemisches Lokalelement mit dem Material des Elektrodenkörpers bildet. Durch eine solche Materialauswahl wird die Wahrscheinlichkeit korrosiver Prozesse, die zu einer Beschädigung der Elektrode oder des elektrischen Leiters führen, verringert.

Vorteilhafterweise besteht der Elektrodenkörper oder zumindest die Stimulationsoberfläche ganz oder teilweise aus einem biokompatiblen und inerten Werkstoff wie zum Beispiel Platin-Iridium, der, inhärent oder durch eine besondere Beschichtung zum Beispiel mit Iridiumoxid oder Graphen, eine hohe Ladungsinjektionskapazität aufweist.

Vorteilhaft ist es, wenn die Fixierungsstruktur des Elektrodenkörpers eine Isolierstoffhülse aufweist, die zur Isolierung eines mechanischen Befestigungselements gegenüber dem Elektrodenkörper eingerichtet ist. Die Isolierstoffhülse bewirkt somit eine elektrische Isolierung eines mechanischen Befestigungselements wie beispielsweise einer Schraube, sodass keine unerwünschten elektrischen Ströme von dem Elektrodenkörper zu dem Befestigungselement erzeugt werden.

Die Fixierungsstruktur des Elektrodenkörpers kann Fixiermittel an dem Umfang oder einem Ende des Elektrodenkörpers oder auch neben dem oder angrenzend an den Elektrodenkörper in das Trägermaterial eingebettete Fixiermittel aufweisen, um den Elektrodenkörper mit dem Gewebe eines Lebewesens zu verbinden.

In einer günstigen Ausführungsform weist die Fixierungsstruktur wenigstens eine Befestigungsöffnung in dem Elektrodenkörper auf, durch die der Elektrodenkörper durch das mechanische Befestigungselement, z.B. eine Schraube, einen Nagel, eine Klammer, einen Faden oder ein ähnliches mechanisches Befestigungselement, am Gewebe des Lebewesens befestigbar ist. Zudem ist es besonders vorteilhaft, wenn die vorbeschriebene Isolierstoffhülse in der Befestigungsöffnung angeordnet ist. Über die Befestigungsöffnung kann dann der Elektrodenkörper beispielsweise an einer Knochenstruktur des Lebewesens befestigt werden, indem ein mechanisches Befestigungselement, wie zum Beispiel eine Schraube, durch die Befestigungsöffnung, besonders vorteilhaft durch eine in der Befestigungsöffnung angeordnete Isolierstoffhülse, geführt und an der Knochenstruktur fixiert wird. Es sind aber auch andere Befestigungsmittel wie chirurgische Fäden zur Befestigung des Elektrodenkörpers denkbar. In diesem Fall wird die Befestigungsöffnung, vorteilhafterweise auch eine in der Befestigungsöffnung angeordnete Isolierstoffhülse so ausgeführt, dass eine Befestigung des alternativen Befestigungsmittels möglich ist. Beispielsweise weist die Isolierstoffhülse im Fall des chirurgischen Fadens mindestens zwei Öffnungen auf.

Gemäß einer vorteilhaften Ausführungsform hat die Isolierstoffhülse eine zweite zumindest teilweise umlaufende Dichtlippe. Dies hat den Vorteil, dass der Elektrodenkörper gegenüber Flüssigkeiten geschützt wird, die durch Lücken zwischen dem Elektrodenkörper oder der den Elektrodenkörper aufnehmenden Elektrodenanordnung und der Isolierstoffhülse zu dem Elektrodenkörper gelangen können. Es ist denkbar, dass die Isolierstoffhülse oder die zweite umlaufende Dichtlippe in einem Stück aus dem Trägermaterial geformt sein kann.

Vorteilhaft ist es, wenn die Isolierstoffhülse zumindest teilweise aus einem Polyetheretherketon (PEEK) besteht. Polyetheretherketon ist ein hochtemperaturbeständiger thermoplastischer Kunststoff, wobei dieser auch in der Medizintechnik zur Anwendung kommt. Polyetheretherketon hat den Vorteil, dass dieser biokompatibel sowie röntgendurchlässig ist. Zudem hat das Material einen geringen Reibungskoeffizienten, sodass nur eine geringe Kraftübertragung von einem Befestigungsmittel wie einer Schraube auf die Isolierstoffhülse und damit auf den Elektrodenkörper erfolgen kann und diesen damit vor Beschädigung wie zum Beispiel einer Deformation schützt. Polyetheretherketon wirkt auch elektrisch isolierend, sodass keine unerwünschten elektrischen Ströme zwischen dem Elektrodenkörper und dem mechanischen Befestigungsmittel fließen können.

Gemäß einer vorteilhaften Ausgestaltung der Erfindung weist der Elektrodenkörper eine vorgeformte konkave Stimulationsoberfläche auf. Eine solche Stimulationsoberfläche kann sich der äußeren Form der meisten Schädelregionen eines Lebewesens optimal anpassen. Der Elektrodenkörper ist somit besser auf die Krümmung der Knochenstruktur abgestimmt.

Alternativ kann der Elektrodenkörper eine vorgeformte ebene Stimulationsoberfläche mit wenigstens einem Einschnitt und/oder einem Ausschnitt aufweisen, durch den eine flexible Anpassbarkeit des Elektrodenkörpers an die Außenkontur des Schädels eines Lebewesens verbessert ist.

Günstig ist es, wenn der Elektrodenkörper zusätzlich geeignet ist, bioelektrische Signale des Lebewesens zu erfassen. Beispielsweise kann mit Hilfe des Elektrodenkörpers ein Elektroenzephalogramm (EEG) oder Elektrokardiogramm (EKG) oder Elektromyogramm (EMG) aufgenommen werden. Hierzu können ein Verstärker mit Analog- und Digitalwandler (ADC) sowie gegebenenfalls ein analoger oder digitaler Signalfilter mit dem Elektrodenkörper verbunden oder in diesen integriert sein, die im Anschluss an die Erfassung bioelektrischer Signale des Lebewesens zu diagnostischen Zwecken oder zur Steuerung der Stimulation verwendbar sind. Zu diesem Zweck kann überdies eine Auswerte- und Steuereinheit mit dem Elektrodenkörper verbindbar oder in eine an den Elektrodenkörper anschließbare Energiequelle integriert sein.

In einer vorteilhaften Ausführungsform hat der Elektrodenkörper einen Flächeninhalt von maximal 7,1 cm², insbesondere einen Flächeninhalt von maximal 4,6 cm². Ein solcher, vergleichsweise kleingebauter Elektrodenkörper ermöglicht eine gute Integration und einen verringerten Eingriff in das Gewebe des Lebewesens, sodass das Risiko einer Gewebeschädigung verringert ist.

Gemäß einer vorteilhaften Ausführungsform weist der Elektrodenkörper eine Dicke von maximal 2 mm, insbesondere eine Dicke von maximal 1 mm auf. Auf diese Weise kann eine mit einem oder mehreren Elektrodenkörpern gebildete Anordnung ebenfalls eine geringe Dicke, beispielsweise von weniger als 3 mm, weniger als 2 mm oder weniger als 1 mm, aufweisen. Auf diese Weise steht eine implantierte Elektrodenanordnung unter der Kopfhaut des Lebewesens kaum hervor. Dies hat einen kosmetischen Vorteil, wobei die Umwelt die implantierte Elektrodenanordnung nicht wahrnehmen kann. Zudem ist die Wahrscheinlichkeit einer Beschädigung durch äu-ßere Einflüsse verringert, da die potentielle Fläche für derartige Beschädigungen verkleinert ist. Auch wird der Tragekomfort für das Lebewesen durch eine geringere Dicke verbessert.

Die Aufgabe wird weiterhin durch eine Elektrodenanordnung mit den Merkmalen des Anspruchs 9 gelöst.

Somit ist eine Elektrodenanordnung zur elektrischen Stimulation von Gewebe eines Lebewesens, insbesondere eine Elektrodenanordnung für die Neurostimulation vorgesehen, mit wenigstens einer Primärelektrode und wenigstens einer Sekundärelektrode. Hierbei sind eine, mehrere oder alle Primärelektroden jeweils durch einen Elektrodenkörper gebildet und/oder eine, mehrere oder alle Sekundärelektroden sind jeweils durch einen Elektrodenkörper gebildet.

Die wenigstens eine Primärelektrode und die wenigstens eine Sekundärelektrode können jeweils mittels elektrischer Leiter mit einem Anschlusselement verbunden sein, wobei das Anschlusselement zum Anschluss an eine andere Elektrode oder eine Energiequelle eingerichtet ist. Erfindungsgemäß sind die Primärelektrode und die Sekundärelektrode an einem gemeinsamen Trägermaterial angeordnet.

Die Elektroden der Elektrodenanordnung sind mit einem Anschlusselement verbunden, wobei das Anschlusselement an eine Energiequelle und/oder an eine elektrische Signalquelle angeschlossen werden kann, um die Elektroden mit Strom zu versorgen. Die elektrische Signalquelle kann zum Senden von elektrischen Stimulationssignalen eingerichtet sein. Die Elektroden können dabei in dem Trägermaterial eingebettet sein. Das Anschlusselement kann dabei einen Steckverbinder aufweisen oder als Steckverbinder ausgebildet sein. Es ist aber auch denkbar, dass das Anschlusselement als Polyetheretherketon-Adapter ausgebildet ist, der den Übergang zwischen der Elektrodenanordnung und der Energiequelle bzw. der elektrischen Signalquelle darstellt. Die Energiequelle und/oder die Signalquelle kann dabei an einer anderen Position im Körper des Lebewesens als die Elektrodenanordnung implantiert werden, wobei die Energiequelle mittels eines elektrischen Leiters mit dem Anschlusselement verbunden und dieser elektrische Leiter mit dem Anschlusselement verschweißt oder verlötet sein kann.

Die erfindungsgemäße Elektrodenanordnung kann dazu eingerichtet sein, zwischen Kopfhaut und Schädel des Lebewesens implantiert zu werden. Gerade im Bereich der Neurostimulation für Epilepsie-Patienten, bei denen eine Behandlung mit Medikamenten nicht anschlägt, wird bisher oft eine maximalinvasive Methode durch Implantation eines intrakraniellen Elektrodensystems angewandt, um den jeweiligen Patienten Linderung zu verschaffen. Die erfindungsgemäße Elektrodenanordnung kann auf dem Schädelknochen des Lebewesens implantiert werden, um die nötige Stimulation bestimmter Gehirnregionen zu erreichen, was einen deutlich weniger invasiven Eingriff bei einem Patienten darstellt und damit eine geringere Belastung für diesen bedeutet.

Die Elektrodenanordnung kann eine Gesamtgröße der Stimulationsoberfläche der Primärelektroden und/oder eine Gesamtgröße der Stimulationsoberfläche aller Sekundärelektroden mit einem Flächeninhalt von wenigstens 50 mm² aufweisen. Das von der Elektrodenanordnung erzeugte elektrische Feld wird von dem Schädel des Lebewesens abgeschwächt. Indem durch eine wenigstens 50 mm² große Stimulationsoberfläche der Elektrodenanordnung ein ausreichend starkes elektrisches Feld erzeugt wird, ohne die Stimulationsoberfläche durch elektrochemische Prozesse zu beschädigen, kann diese Abschwächung durch den Schädel besser kompensiert werden. Des Weiteren erweist sich die mindestens 50 mm² große Stimulationsoberfläche als vorteilhaft, wenn elektrische Pulse mit einer langen Pulsdauer verwendet werden, da sie bei einer konstanten Stromstärke zu geringeren Stromdichten an der Stimulationsoberfläche führen und somit ebenfalls irreversible elektrochemische Prozesse verringert werden. Überdies wird die potentiell stimulierbare Hirnfläche vergrö-ßert. Die vergleichsweise große Stimulationsoberfläche wirkt sich auch günstig auf das Signal-zu-Rausch-Verhältnis aus. Die Elektrodenfläche ist dabei nach oben durch die Gesamtgröße der Elektrodenanordnung begrenzt, um die Elektrodenanordnung beim Menschen noch implantieren zu können.

Alternativ oder zusätzlich kann die Elektrodenanordnung eine Gesamtgröße der Stimulationsoberfläche der Primärelektroden und/oder eine Gesamtgröße der Stimulationsoberfläche aller Sekundärelektroden mit einem Flächeninhalt von wenigstens 20 mm² und zusätzlich eine die wirksame Stimulationsoberfläche vergrößernde Oberflächenbehandlung aufweisen. Beispielsweise kann bei einer solchen Oberflächenbehandlung eine rauere oder genoppte Oberfläche oder auch eine fraktale Oberfläche erzeugt werden, wodurch sich eine Oberflächenvergrößerung ergibt und die Übergangsimpedanz zwischen Elektrodenkörper und Gewebe verringert wird, sodass das Signal-zu-Rausch-Verhältnis verbessert wird.

Alternativ oder zusätzlich weist die Elektrodenanordnung eine Fixierungsstruktur für die Fixierung der Elektrodenanordnung am Gewebe eines Lebewesens auf.

Alternativ oder zusätzlich ist die Elektrodenanordnung bis auf die Stimulationsoberflächen der Primär- und Sekundärelektroden von einem elektrisch isolierenden Trägermaterial vollständig umgeben. Dies ermöglicht die einfache Anordnung und Fixierung der Elektrodenanordnung auf dem Gewebe des Lebewesens. Zudem wird durch das elektrisch isolierende Trägermaterial das Auftreten von Leck- und Kurzschlussströmen an den Elektroden verhindert oder zumindest reduziert. Hierbei kann die Elektrodenanordnung auch von mehreren elektrisch isolierenden Trägermaterialien umgeben sein. Beispielsweise kann auf die Primär- und/oder Sekundärelektroden ein Polyimid-Substrat aufgebracht sein, das zusätzlich in Silikon eingebettet sein kann.

In einer vorteilhaften Ausgestaltung der Elektrodenanordnung ist an dem Trägermaterial ein Verstärkungselement, insbesondere ein Verstärkungsnetz, angeordnet. Durch ein derartiges Verstärkungselement kann die Reißfestigkeit der Elektrodenanordnung erhöht werden. Dies hat den Vorteil, dass die geometrische Genauigkeit der Elektrodenanordnung, d.h. die Lage der Primärelektrode und der Sekundärelektroden zueinander, und damit das notwendige elektrische Feld gewährleistet werden kann. Vorteilhafterweise ist das Verstärkungselement ein Verstärkungsnetz wie zum Beispiel ein chirurgisches Netz, dessen Stränge sich zwischen der Primärelektrode und den Sekundärelektroden erstrecken. Dabei ist es möglich, dass bestimmte Teile der Elektrodenanordnung kein Verstärkungselement haben. So ist zum Beispiel denkbar, dass an dem Kontaktierungsabschnitt der Elektrodenanordnung kein derartiges Verstärkungselement angeordnet ist, um dessen Flexibilität nicht zu verringern.

Gemäß einer vorteilhaften Ausführungsform der Elektrodenanordnung ist zumindest ein elektrischer Leiter zumindest teilweise vom Trägermaterial umgeben. Dies hat den Vorteil, dass der elektrische Leiter an dem Trägermaterial in seiner Position gehalten wird. Dies vermeidet eine unerwünschte Verlagerung des elektrischen Leiters. Zudem wird der elektrische Leiter gegen äußere Einflüsse isoliert, sodass dieser zum Beispiel vor Flüssigkeiten abgeschirmt und damit eine unerwünschte Reaktion verhindert wird.

Günstig ist es, wenn die Dicke der Elektrodenanordnung kleiner als 4 mm, insbesondere kleiner als 2 mm, ist. Auf diese Weise steht eine implantierte Elektrodenanordnung unter der Kopfhaut des Lebewesens kaum hervor. Dies hat einen kosmetischen Vorteil, wobei die Umwelt die implantierte Elektrodenanordnung nicht wahrnehmen kann. Zudem ist die Wahrscheinlichkeit einer Beschädigung durch äußere Einflüsse verringert, da die potentielle Fläche für derartige Beschädigungen verkleinert ist. Auch wird der Tragekomfort für das Lebewesen durch eine geringere Dicke verbessert.

Gemäß einer vorteilhaften Ausführungsform weist die Elektrodenanordnung wenigstens eine Primärelektrode und wenigstens zwei Sekundärelektroden auf. Besonders vorteilhaft ist es, wenn die Elektrodenanordnung mindestens vier Sekundärelektroden aufweist. Eine höhere Anzahl von Elektroden lässt eine genauere Steuerung der elektrischen Feldkonfiguration zu, was zum Beispiel aufgrund von Inhomogenitäten der Leitfähigkeit des Gewebes, oder zur genaueren Fokussierung des elektrischen Feldes auf ein Gewebeareal von Vorteil sein kann. Zudem kann bei mehreren Elektroden zwischen mehreren Kanälen einer Signalableitung gewählt werden, was den Informationsgehalt erhöht. Vorteilhaft ist es, wenn die Elektrodenanordnung zwischen drei und acht Sekundärelektroden hat, insbesondere genau vier Sekundärelektroden.

Vorteilhaft ist es, wenn wenigstens einer der elektrischen Leiter der Sekundärelektroden mäanderförmig um die Primärelektrode angeordnet ist, wobei die mäanderförmige Anordnung des elektrischen Leiters entlang einer gebogenen Bahn um die Primärelektrode verläuft. Eine mäanderförmige Anordnung verringert die mechanische Belastung der elektrischen Leiter, insbesondere deren Anschlussbereiche, die zum Beispiel durch Biegen und Ziehen der Elektrodenanordnung entstehen kann. Durch die Anordnung auf einer gebogenen Bahn wird die Entlastung weiterhin verbessert. Vorteilhafterweise verläuft die gebogene Bahn über einen Winkelbereich von 40° bis 100°, ausgehend von einem 360°-System, um die Primärelektrode.

Grundsätzlich kann das Trägermaterial der Elektrodenanordnung Befestigungsstrukturen aufweisen, mit denen die Elektrodenanordnung an einer Knochenstruktur des Lebewesens befestigbar ist. Die Befestigungsstrukturen können beispielsweise als Schraublöcher oder Ösen zur Herstellung einer Nahtverbindung ausgebildet sein.

Alternativ oder in Kombination haben die Primärelektrode und/oder zumindest eine der Sekundärelektroden jeweils wenigstens eine Befestigungsöffnung, wobei eine Isolierstoffhülse in jeweils einer der Befestigungsöffnungen angeordnet ist. Über die Befestigungsöffnung kann dann die Elektrodenanordnung an einer Gewebestruktur des Lebewesens befestigt werden, indem Befestigungsmittel wie zum Beispiel Schrauben durch die in den Befestigungsöffnungen angeordneten Isolierstoffhülsen geführt werden und an der Gewebestruktur fixiert werden. Es sind aber auch andere Befestigungsmittel wie chirurgische Fäden zur Befestigung der Elektroden denkbar. In diesem Fall wird die Isolierstoffhülse so ausgeführt, dass eine Befestigung des alternativen Befestigungsmittels möglich ist, im Fall des chirurgischen Fadens zum Beispiel durch mindestens zwei Öffnungen in der Isolierstoffhülse. Durch die Festlegung über die Primärelektrode und/oder die Sekundärelektroden können gleichzeitig die übrigen Elektroden an einer gewünschten Position gehalten und ein verbesserter Kontakt zum Schädelknochen erreicht werden, sodass das gewünschte elektrische Feld auch über die Implantationszeit der Elektrodenanordnung an der gewünschten Stelle erzeugt werden kann. Eine derartige Befestigung vermindert zudem unerwünschten Gewebeeinwuchs zwischen Elektrode und Schädelknochen. Weiterhin bewirkt die Isolierstoffhülse eine elektrische Isolierung des Befestigungsmittels, sodass keine unerwünschten elektrischen Ströme von der Primärelektrode und/oder den Sekundärelektroden zu dem Befestigungsmittel erzeugt werden.

Die Isolierstoffhülse kann zumindest teilweise aus einem Polyetheretherketon (PEEK) bestehen. Polyetheretherketon ist ein hochtemperaturbeständiger thermoplastischer Kunststoff, wobei dieser auch in der Medizintechnik zur Anwendung kommt. Polyetheretherketon hat den Vorteil, dass dieser biokompatibel sowie röntgendurchlässig ist. Zudem hat das Material einen geringen Reibungskoeffizienten, sodass nur eine geringe Kraftübertragung von der Schraube auf die Isolierstoffhülse und damit auf die jeweilige Elektrode erfolgen kann und diese damit vor Beschädigung wie zum Beispiel einer Deformation schützt. Polyetheretherketon wirkt auch elektrisch isolierend, sodass keine unerwünschten elektrischen Ströme zwischen den Elektroden und der Schraube fließen können.

Die Isolierstoffhülse der Elektrodenanordnung kann eine zweite zumindest teilweise umlaufende Dichtlippe haben. Dies hat den Vorteil, dass die Elektroden gegenüber Flüssigkeiten, die durch Lücken zwischen der Elektrodenanordnung und der Isolierstoffhülse zu den Elektroden gelangen können, geschützt werden. Es ist denkbar, dass die Isolierstoffhülse oder die zweite umlaufende Dichtlippe in einem Stück aus dem Trägermaterial geformt sein kann.

Erfindungsgemäß haben die Primärelektrode und/oder zumindest eine der Sekundärelektroden über den Umfang verteilt angeordnete Ausnehmungen zur Fixierung im Trägermaterial. Derartige Ausnehmungen haben den Vorteil, dass die Primärelektrode und die Sekundärelektroden besser in dem Trägermaterial fixiert gehalten werden können, indem eine verbesserte formschlüssige Verbindung erreicht werden kann. Bei der Herstellung gelangt das zunächst flüssige Trägermaterial wie zum Beispiel ein Silikon in die Ausnehmungen der Primärelektrode und der Sekundärelektroden, wobei nach Aushärten des Trägermaterials die Primärelektrode und die Sekundärelektroden fest mit dem Trägermaterial verbunden sind. Die Ausnehmungen können zum Beispiel ringförmig um die Befestigungsöffnung der Primärelektrode und/oder zumindest einer der Sekundärelektroden und/oder entlang des Elektrodenrands der Primärelektrode und/oder zumindest einer der Sekundärelektroden angeordnet sein.

Alternativ oder in Kombination ist eine Verbindungsstelle des elektrischen Leiters mit der Primärelektrode und/oder mit zumindest einer der Sekundärelektroden ein tangentialer Leiteranschluss. Ein tangentialer Leiteranschluss verläuft dabei orthogonal zur Verbindungslinie zwischen Berührungspunkt und Mittelpunkt der jeweiligen Elektrode. Der Leiter berührt die Elektrode wie eine Tangente. Dies hat den Vorteil, dass die Verbindungsstelle bei Belastung des elektrischen Leiters wie zum Beispiel durch eine Zugkraft entlastet wird, sodass die Wahrscheinlichkeit einer Beschädigung der Verbindungsstelle und damit eines Ausfalls der jeweiligen Elektrode verringert wird. Der elektrische Leiter kann dabei zum Beispiel an die Elektrode angeschweißt oder aufgelötet werden.

Es ist jedoch auch denkbar, dass eine Verbindungsstelle des elektrischen Leiters mit der Primärelektrode und/oder mit zumindest einer der Sekundärelektroden als ein rechtwinkliger Leiteranschluss ausgebildet ist. Unter einem rechtwinkligen Leiteranschluss wird ein Leiteranschluss verstanden, der die Kontur- oder Umfangslinie der Elektrode im Anschlussbereich senkrecht schneidet, sodass sich ein rechter Winkel zwischen dem Leiter und der Kontur- oder Umfangslinie im Anschlussbereich ausbildet. Im Vergleich zum tangentialen Leiteranschluss verläuft der rechtwinklige Leiteranschluss parallel statt orthogonal zur Verbindungslinie zwischen Berührungspunkt und Mittelpunkt der Elektrode. Diese Leiteranschlussform ist beispielsweise bei Polyimid-Substraten vorteilhaft.

In einer günstigen Ausführungsform hat die Elektrodenanordnung einen Kontaktierungsabschnitt, wobei das Anschlusselement an dem Kontaktierungsabschnitt angeordnet ist und wobei der Kontaktierungsabschnitt seitlich von der Elektrodenanordnung abragt. Durch den abragenden Kontaktierungsabschnitt kann das Anschlusselement aus dem Bereich der Primärelektrode und der Sekundärelektrode mechanisch isoliert werden, sodass unerwünschte Wechselwirkungen vermieden werden.

Ferner ist es vorteilhaft, wenn der Kontaktierungsabschnitt eine größere Länge als Breite hat. Die Länge ist die Dimension in Abragerichtung des Kontaktierungsabschnittes, wobei die Breite die Dimension quer zur Abragerichtung des Kontaktierungsabschnittes ist. Durch die längliche Ausgestaltung können auf den Kontaktierungsabschnitt einwirkende Kräfte abgetragen werden, sodass diese Kräfte nicht in den Bereich der Elektroden gelangen und dort zu Beschädigungen führen können.

Weiter ist es vorteilhaft, wenn an dem Kontaktierungsabschnitt Befestigungselemente zur Schraubbefestigung angeordnet sind. Die Befestigungselemente können zum Beispiel als Langlöcher ausgebildet sein, wobei der Kontaktierungsabschnitt mittels Schrauben oder anderer Befestigungsmittel an dem Lebewesen befestigt werden kann. Auf diese Weise kann eine sichere Befestigung des Kontaktierungsabschnittes an dem Lebewesen erfolgen, wobei mechanische Kräfte über den Schädel abgetragen werden können. Die Befestigungselemente können derartig angeordnet sein, dass diese ein sechswertiges Lager bilden, wobei die Rotations- und Translationsfreiheitsgrade beschränkt werden.

Gemäß einer günstigen Ausführungsform ist die Gesamtfläche der Sekundärelektroden größer als die Fläche der Primärelektrode. Diese Ausführungsform ermöglicht eine gute Stromverteilung über die Gewebefläche des Lebewesens und ein verringertes Risiko einer Gewebeschädigung. Ferner ist es vorteilhaft, wenn die Primärelektrode und/oder die Sekundärelektroden jeweils eine Fläche von 50 mm² bis 71 mm², insbesondere eine Fläche von 20 mm² bis 46 mm² haben. Mit diesen Elektrodenflächen wird eine besonders gute Stromverteilung bei verringertem Risiko einer Gewebeschädigung erzielt.

Alternativ kann die Gesamtfläche der Sekundärelektroden in einer vorteilhaften Ausgestaltung auch kleiner sein als die Fläche der Primärelektrode. Diese Ausgestaltung erlaubt eine umgekehrte Stimulationsstromrichtung wie eine anodale Stimulation statt einer kathodalen Stimulation, ohne zu einer korrosionsbedingten Schädigung der Elektroden zu führen. Hierdurch können unterschiedliche Indikationen und Krankheitsbilder therapiert werden.

Vorteilhaft ist es, wenn die Sekundärelektroden auf wenigstens einer Kreisbahn um die Primärelektrode angeordnet sind. In einer weiter vorteilhaften Ausgestaltung können die Sekundärelektroden der Elektrodenanordnung äquidistant auf wenigstens einer Kreisbahn um die Primärelektrode angeordnet sein. Zudem ist es günstig, wenn die Sekundärelektroden auf einer konzentrischen Kreisbahn um die Primärelektrode verteilt sind. Diese Verteilung der Sekundärelektroden um die Primärelektrode begünstigt die Generierung eines elektrischen Feldes durch eine Abschirmung wie zum Beispiel eine Schädeldecke hindurch zusätzlich.

Gemäß einer günstigen Ausführungsform ist der Mittelpunkt wenigstens einer der Sekundärelektroden in einem Abstand von 5 mm bis 55 mm, insbesondere in einem Abstand von 10 mm bis 50 mm, zum Mittelpunkt der Primärelektrode angeordnet. Ferner ist es vorteilhaft, wenn der Rand wenigstens einer der Sekundärelektroden in einem Abstand von 1 mm bis 8 mm, insbesondere in einem Abstand von 2 mm bis 6 mm, zum Rand der Primärelektrode angeordnet ist. Es hat sich gezeigt, dass geringe Abstände der Sekundärelektroden zu der Primärelektrode eine optimale Fokussierung des elektrischen Feldes ermöglichen, wobei durch einen ausreichenden Abstand der Sekundärelektroden zu der Primärelektrode die Wahrscheinlichkeit eines Leck- und Kurzschlussstroms verringert bzw. ein solcher verhindert sowie die Eindringtiefe des elektrischen Feldes in den Kopf vergrößert werden kann. Diese Abstände bilden damit einen optimierten Mittelweg zwischen den gegenläufigen Anforderungen der Fokussierung und der Leck- bzw. Kurzschlussströme. Ein größerer Abstand der Elektroden führt zu einem höheren Signal, aber auch zu einer geringeren Fokalität.

Vorteilhafterweise hat das Trägermaterial wenigstens eine Aussparung, wobei die Aussparung jeweils zwischen benachbarten Sekundärelektroden angeordnet ist. Es ist auch denkbar, dass das Trägermaterial abschnittsweise eine Verjüngung, also eine abnehmende oder geringere Materialstärke aufweist. Auf diese Weise kann die Elektrodenanordnung flexibel ausgestaltet sein. Die Aussparung oder Verjüngung erhöht die Flexibilität insbesondere an den Sekundärelektroden, wobei vorteilhafterweise zwischen jeweils zwei benachbarten Sekundärelektroden eine Aussparung oder Verjüngung angeordnet ist. Durch die erhöhte Flexibilität kann so auf die unregelmäßige Oberfläche der Schädeldecke eines Lebewesens reagiert werden, indem die Elektrodenanordnung an jeder Position einen ausreichenden Kontakt zur Schädeldecke hat.

Es wird vorgeschlagen, dass das Trägermaterial eine erste zumindest teilweise umlaufende Dichtlippe an einer freiliegenden Kontaktierungsseite der Primärelektrode und/oder an zumindest einer der Sekundärelektroden hat. Eine umlaufende Dichtlippe hat den Vorteil, dass die Elektroden vor unerwünschten Wechselwirkungen an der implantierten Stelle mit der Umgebung geschützt werden, wobei die umlaufende Dichtlippe eine Isolierung bereitstellt, die auch bei Deformation der Elektrodenanordnung intakt bleibt. Im Gegensatz zu aus dem Stand der Technik bekannten Elektrodenanordnungen kann so die Haltbarkeit verbessert sowie Kurzschlüsse durch Elektrolyte oder Gewebe, das sich zwischen Elektrode und Schädel befindet, verhindert oder zumindest deutlich reduziert werden. Weiterhin wird das Signal-zu-Rausch-Verhältnis verbessert. Es ist denkbar, dass die erste umlaufende Dichtlippe in einem Stück aus dem Trägermaterial geformt sein kann.

Gemäß einer vorteilhaften Ausführungsform überragt eine erste zumindest teilweise umlaufende Dichtlippe zumindest teilweise eine, mehrere oder alle Primärelektroden und/oder zumindest teilweise eine, mehrere oder alle Sekundärelektroden um 0,05 mm bis 1 mm, insbesondere 0,1 mm bis 0,3 mm. Es hat sich gezeigt, dass diese Abmessungen die Flexibilität der Elektrodenanordnung gewährleisten und gleichzeitig eine effiziente Isolierung der Elektroden vor äußeren Einflüssen bereitstellen. Die Dichtlippe kann den Elektrodenkörper der jeweiligen Elektrode dabei in jeder Raumrichtung überragen. Beispielsweise kann die Dichtlippe den Elektrodenkörper in Normalenrichtung der Stimulationsoberfläche und/oder zentripetal zu der Stimulationsoberfläche überragen.

Jede in dieser Anmeldung beschriebene Isolierung, beispielsweise durch das Trägermaterial oder Dichtlippen, trägt auch zu einer verbesserten Signalqualität der Elektroden bei, sodass das Signal-zu-Rausch-Verhältnis verbessert wird.

Die Verbindungsstelle des elektrischen Leiters mit der Primärelektrode und/oder zumindest einer der Sekundärelektroden kann abgedichtet sein, insbesondere mit einem Epoxidharz oder einem anderen Dichtmittel. Dies hat den Vorteil, dass die empfindliche Verbindungsstelle vor äußeren Einflüssen wie zum Beispiel Flüssigkeiten geschützt wird, die zu einem Ausfall, zum Beispiel durch Korrosion, der jeweiligen Elektrode führen würde.

Vorteilhafterweise ist der elektrische Leiter aus einem Material gefertigt, welches, insofern es in elektrischem Kontakt mit dem Elektrodenmaterial steht, in wässriger Elektrolytlösung kein elektrochemisches Lokalelement bildet. Durch eine solche Materialauswahl wird die Wahrscheinlichkeit korrosiver Prozesse, die zu einer Beschädigung der Elektroden oder des elektrischen Leiters führen, verringert.

Ferner ist ein Verfahren zur Herstellung einer Elektrodenanordnung zur elektrischen Stimulation von Gewebe eines Lebewesens möglich, insbesondere zur Herstellung einer Elektrodenanordnung gemäß den vorbeschriebenen Merkmalen.

In einem Verfahrensschritt a) werden, z.B. durch Laserschneiden oder Stanzen, Elektrodenkörper aus einer Folie eines Elektrodenmaterials mit geeigneter Dicke hergestellt. In einem Verfahrensschritt b) wird gegebenenfalls eine Oberflächenbeschichtung und/oder eine Strukturierung der Oberfläche auf zumindest einen Teil der Elektrodenfläche aufgebracht. In einem Verfahrensschritt c) wird an jedem Elektrodenkörper ein elektrischer Leiter angebracht oder der elektrische Leiter aus einem Stück zusammen mit den Elektroden hergestellt. In einem Verfahrensschritt d) wird gegebenenfalls ein Epoxidharz oder ein anderer elektrischer Isolator auf die Verbindungsstelle aufgebracht. In einem Verfahrensschritt e) wird eine Form der Elektrodenanordnung aus dem Trägermaterial hergestellt. In einem Verfahrensschritt f) wird der Elektrodenkörper sowie gegebenenfalls der elektrische Leiter und gegebenenfalls das Verstärkungselement eingebettet. In einem Verfahrensschritt g) wird ein elektrischer Leiter am Kontaktierungsabschnitt der Elektrodenanordnung angeschlossen.

Ferner ist ein weiteres Verfahren zur Herstellung einer Elektrodenanordnung zur elektrischen Stimulation von Gewebe eines Lebewesens möglich, insbesondere zur Herstellung einer Elektrodenanordnung gemäß den vorbeschriebenen Merkmalen.

In einem Verfahrensschritt a) wird ein Trägermaterial bereitgestellt. In einem Verfahrensschritt b) wird ein oder werden mehrere Elektrodenkörper auf dem Trägermaterial durch einen Gasphasenabscheideprozess und/oder durch galvanische Abscheidung erzeugt. In einem Verfahrensschritt c) wird gegebenenfalls eine Oberflächenbeschichtung und/oder Strukturierung einer Oberfläche auf zumindest einen Teil der Elektrodenfläche aufgebracht. In einem Verfahrensschritt d) wird gegebenenfalls das Trägermaterial in ein weiteres isolierendes Trägermaterial eingebettet.

In einer vorteilhaften Ausführungsform der Verfahren wird wenigstens ein elektrischer Leiter zur elektrischen Verbindung des Elektrodenkörpers mit einem elektrischen Gerät oder einem anderen Elektrodenkörper durch einen Gasphasenabscheideprozess und/oder durch galvanische Abscheidung erzeugt und an den Elektrodenkörper angeschlossen.

Die Erfindung wird nachfolgend beispielhaft mit den beigefügten Zeichnungen näher erläutert. Es zeigen:
- Figur 1 -: einen schematischen Aufbau eines Elektrodenkörpers;
- Figur 2 -: eine schematische Darstellung eines Elektrodenkörpers mit einem angeschlossenen elektrischen Leiter und einer Isolierstoffhülse;
- Figur 3 -: eine schematische Darstellung des Elektrodenkörpers aus Figur 2 mit einem Trägermaterial;
- Figur 4 -: eine schematische Darstellung mehrerer Elektrodenkörper, die über elektrische Leiter an eine Energiequelle angeschlossen sind;
- Figur 5 -: eine schematische Darstellung eines in ein Trägermaterial eingebetteten Elektrodenkörpers in einer Seitenansicht;
- Figur 6 -: eine schematische Darstellung einer Elektrodenanordnung in einer Draufsicht;
- Figur 7 -: eine schematische Darstellung einer Elektrodenanordnung mit in ein Trägermaterial eingebetteten Elektroden in einer Seitenansicht;
- Figur 8 -: eine schematische Darstellung eines Verfahrens zur Herstellung einer Elektrodenanordnung;
- Figur 9 -: eine schematische Darstellung eines weiteren Verfahrens zur Herstellung einer Elektrodenanordnung.

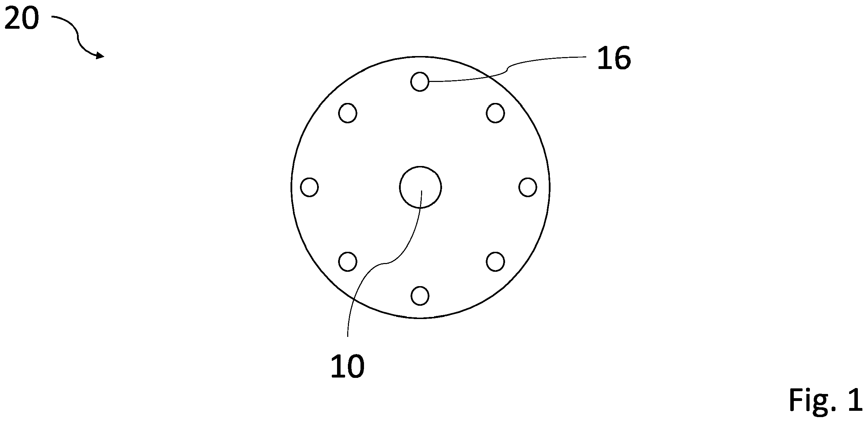

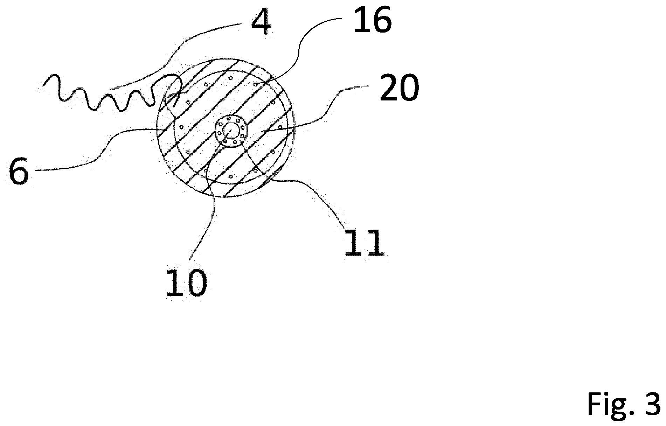

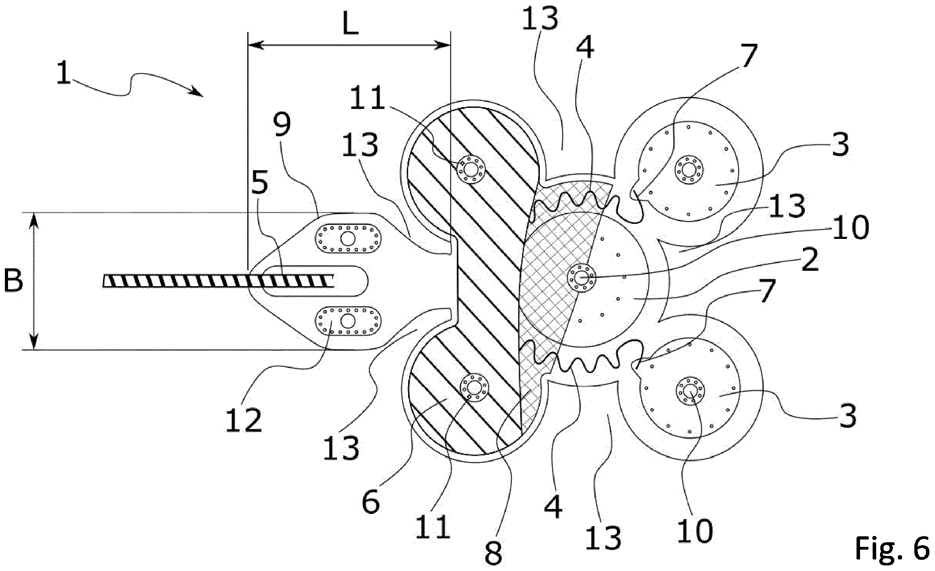

Figur 1 zeigt einen schematischen Aufbau eines Elektrodenkörpers 20. Der dargestellte Elektrodenkörper 20 ist dabei als Rundelektrode ausgeführt. Es sind aber auch andere Elektrodenformen denkbar. Deutlich wird, dass der Elektrodenkörper 20 eine Befestigungsöffnung 10 zur Befestigung an einem Lebewesen hat. Deutlich wird weiterhin, dass der Elektrodenkörper 20 über seinen Umfang verteilt angeordnete Ausnehmungen 16 hat. Durch die Ausnehmungen 16 wird eine verbesserte Fixierung des Elektrodenkörpers 20 in einem beispielsweise in Figur 3 gezeigten Trägermaterial 6 erreicht. Bei der Herstellung einer den Elektrodenkörper 20 aufnehmenden, beispielsweise in Figur 6 gezeigten Elektrodenanordnung 1 ist das Trägermaterial 6 zunächst in einem flüssigen Zustand, wobei das Trägermaterial 6 sich in den Ausnehmungen 16 verteilt und der Elektrodenkörper 20 damit nach dem Aushärten des Trägermaterials 6 fixiert gehalten werden kann.

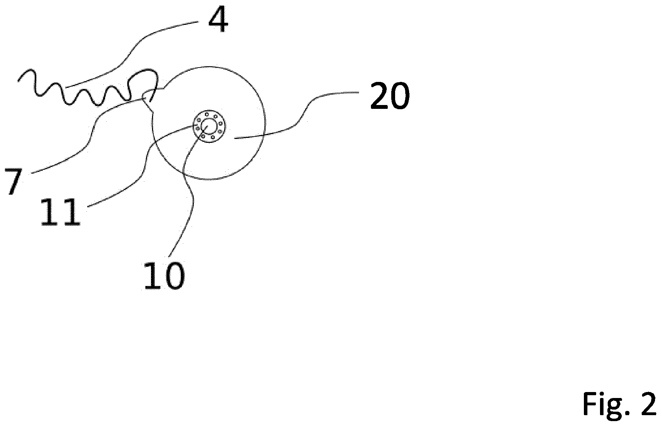

Figur 2 zeigt schematisch einen ebenfalls als Rundelektrode ausgeführten Elektrodenkörper 20 mit einem angeschlossenen elektrischen Leiter 4. Zum Anschluss des elektrischen Leiters 4 ist an dem Elektrodenkörper 20 ein Leiteranschluss 7 vorgesehen, der beispielsweise als aus der Kreisoberfläche der Rundelektrode herausragender Vorsprung ausgebildet sein kann, um die wirksame Stimulationsoberfläche des Elektrodenkörpers 20 nicht zu verringern. Der Leiteranschluss 7 ist vorliegend als tangentialer Leiteranschluss 7 ausgebildet, sodass der angeschlossene elektrische Leiter 4 den Elektrodenkörper 20 wie eine Tangente berührt. Hierdurch wird die Anschlussstelle bei einer auftretenden Zugkraft entlastet und somit eine Beschädigung des Leiteranschlusses 7 mit einem resultierenden Ausfall der Elektrode verhindert.

Der elektrische Leiter 4 kann an den Leiteranschluss 7 des Elektrodenkörpers 20 angelötet oder angeschweißt sein, wobei es vorteilhaft ist, wenn die Verbindungsstelle gegen äußere Einflüsse zum Beispiel mit einem Epoxidharz abgedichtet ist. Zu erkennen ist, dass der elektrische Leiter 4 mäanderförmig verläuft. Dies verringert die Belastung des elektrischen Leiters 4, insbesondere in dessen Anschlussbereich am Leiteranschluss 7, die zum Beispiel durch Biegen und Ziehen einer den Elektrodenkörper aufnehmenden Elektrodenanordnung 1 entstehen können.

Weiterhin ist zu erkennen, dass der Elektrodenkörper 20 eine Befestigungsöffnung 10 aufweist, in die eine Isolierstoffhülse 11 eingesetzt ist. Dadurch kann der Elektrodenkörper 20 über ein Befestigungselement, beispielsweise eine Schraube, durch die Isolierstoffhülse 11 an einem Schädel des Lebewesens befestigt werden. Die Isolierstoffhülse 11 verhindert dabei, dass ein zu großes Drehmoment auf den Elektrodenkörper 20 übertragen wird und diesen damit beschädigt. Des Weiteren wird ein unerwünschter Stromfluss zwischen dem Elektrodenkörper 20 und dem Befestigungselement vermieden. Die Isolierstoffhülse 11 kann dabei zum Beispiel aus einem Polyetheretherketon bestehen. Durch die Befestigung des Elektrodenkörpers 20 über eine Befestigungsöffnung 10 kann eine exakte Lage des Elektrodenkörpers 20 gewährleistet werden, der ein erwünschtes elektrisches Feld in der Zielregion erzeugen kann.

In Figur 3 ist der Elektrodenkörper 20 aus Figur 2 dargestellt. In dieser Ansicht ist zu erkennen, dass der Elektrodenkörper 20 in ein schraffiert angedeutetes Trägermaterial 6 eingebettet ist. Das Trägermaterial 6 ist elektrisch isolierend und umgibt den Elektrodenkörper 20 vollständig bis auf die Stimulationsoberfläche. Das Trägermaterial 6 kann dabei zum Beispiel aus einem Silikon bestehen. Es sind aber auch andere Materialen denkbar, die die anwendungsbedingten Eigenschaften (Biokompatibilität, Flexibilität, elektrisch isolierend, etc.) erfüllen. Das Trägermaterial 6 ermöglicht eine einfache Anordnung und Fixierung des Elektrodenkörpers 20 in einer übergeordneten Struktur, insbesondere einer Elektrodenanordnung 1. Zudem wird durch das elektrisch isolierende Trägermaterial 6 das Auftreten von Leck- und Kurzschlussströmen verhindert oder zumindest reduziert.

Weiterhin weist der Elektrodenkörper 20 Ausnehmungen 16 auf. Das in einem Herstellungsprozess zunächst flüssige Trägermaterial 6 dringt in die Ausnehmungen 16 und unterstützt somit nach dem Aushärten die Fixierung des Elektrodenkörpers 20 in dem Trägermaterial 6.

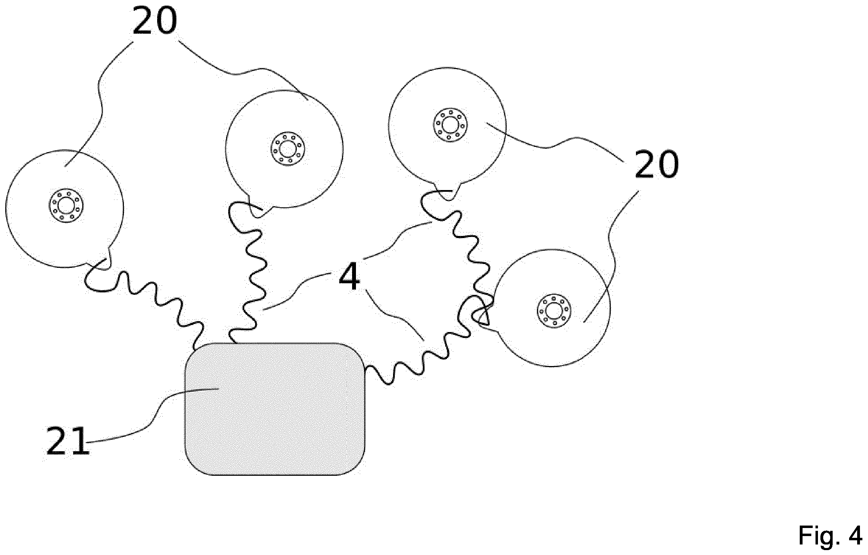

Figur 4 zeigt schematisch mehrere, nämlich insgesamt vier Elektrodenkörper 20, die über elektrische Leiter 4 mit einer Energiequelle 21 verbunden sind. Die Energiequelle 21 kann als Versorgungsquelle ausgebildet sind, um die Elektrodenkörper mit elektrischem Strom zu versorgen und hierdurch ihren Betrieb zu ermöglichen. Alternativ oder zusätzlich kann die Energiequelle 21 als Signalquelle ausgebildet sein, um gezielt elektrische Stimulationssignale an den Elektroden zu erzeugen.

Zu erkennen ist, dass die Elektrodenkörper 20 nicht unmittelbar mit der Energiequelle 21 verbunden sein müssen. Es ist genauso auch eine Anordnung denkbar, in der ein Elektrodenkörper 20 mit einem weiteren Elektrodenkörper 20 verbunden ist, der wiederum an die Energiequelle 21 angeschlossen ist. Hierzu verläuft der jeweilige elektrische Leiter 4 entweder zwischen einem Elektrodenkörper 20 und der Energiequelle 21 oder zwischen zwei Elektrodenkörpern 20. Durch eine mittelbare Verbindung eines Elektrodenkörpers 20 mit der Energiequelle 21 über einen weiteren Elektrodenkörper 20 können auch weiter von der Energiequelle 21 entfernte Geweberegionen erreicht werden, ohne längere elektrische Leiter 4 vorhalten zu müssen oder diese zu strecken und dadurch einem höheren Belastungsrisiko auszusetzen. Selbstverständlich können auch mehrere, also mehr als zwei Elektrodenkörper 20 mit einem weiteren Elektrodenkörper 20 verbunden werden. An dem zur Verbindung der elektrischen Leiter 4 vorgesehenen Leiteranschluss 7 können die mehreren Leiter 4 beispielsweise nebeneinander angelötet werden. Grundsätzlich ist es natürlich ebenfalls denkbar, einen längeren Leiter 4 für den räumlich entfernteren Elektrodenkörper 20 zu verwenden und einen weiteren, räumlich näheren Elektrodenkörper 20 als Abzweig elektrisch anzubinden.

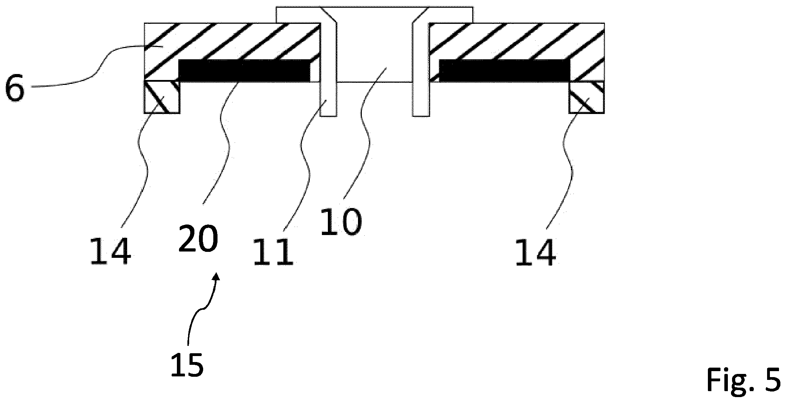

Figur 5 zeigt den in ein Trägermaterial 6 eingebetteten Elektrodenkörper 20 in einer Seitenansicht. Zu erkennen ist, dass der Elektrodenkörper 20 eine durchgehende Befestigungsöffnung 10 hat, wobei in der Befestigungsöffnung 10 eine Isolierstoffhülse 11 angeordnet ist und wobei der Elektrodenkörper 20 mittels eines nicht gezeigten Befestigungselements über die Isolierstoffhülse 11 an einer Knochen- oder anderweitigen Gewebestruktur des Lebewesens befestigt werden kann. Es wird deutlich, dass das Trägermaterial 6 eine erste umlaufende Dichtlippe 14 an einer Kontaktierungsseite 15 des Elektrodenkörpers 20 hat. Die umlaufende Dichtlippe 14 kann dabei einstückig aus demselben Material des Trägermaterials 6 gebildet sein. Auf diese Weise kann der Elektrodenkörper 20 trotz Unebenheiten an der zu implantierenden Stelle befestigt werden, ohne dass es zu Deformationen an dem Elektrodenkörper 20 kommt. Dabei ist es auch denkbar, dass die umlaufende Dichtlippe 14 den Elektrodenkörper 20 teilweise überragt, wodurch eine verbesserte Fixierung des Elektrodenkörpers 20 in dem Trägermaterial 6 ermöglicht wird. Weiterhin erkennbar ist, dass der Elektrodenkörper 20 auf der Kontaktierungsseite 15 freiliegend und auf der anderen Seite vom Trägermaterial 6 isolierend überdeckt ist. Der Elektrodenkörper 20 ist damit im Trägermaterial 6 eingebettet. Dies ermöglicht eine effiziente Isolierung des Elektrodenkörpers 20 nach außen und gewährleistet zugleich die Generierung eines erwünschten elektrischen Feldes in einer Körperregion zur Kontaktierungsseite 15 hin. Es ist auch denkbar, dass die Isolierstoffhülse 11 eine zweite, nicht gezeigte umlaufende Dichtlippe hat, die diesen Effekt noch zusätzlich begünstigt.

Figur 6 zeigt in einer schematischen Darstellung eine Elektrodenanordnung 1 in einer Draufsicht. Zur Verdeutlichung ist die Elektrodenanordnung 1 teilweise freigeschnitten, wobei unterschiedliche Schichten der Elektrodenanordnung 1 zu erkennen sind. Die Elektrodenanordnung 1 hat eine Primärelektrode 2, wobei die Primärelektrode 2 von vier Sekundärelektroden 3 umgeben ist. Die Sekundärelektroden 3 sind dabei auf einer gemeinsamen Kreisbahn um die Primärelektrode 2 angeordnet. Die Primärelektrode 2 und die Sekundärelektroden 3 sind dabei über jeweils einen elektrischen Leiter 4 mit einem Anschlusselement 5 verbunden, wobei das Anschlusselement 5 zum Anschluss an eine Energiequelle 21 eingerichtet ist. Die Primär- und Sekundärelektroden 2, 3 können vorteilhafterweise weitere Merkmale des vorbeschriebenen Elektrodenkörpers 20 aufweisen.

Es wird deutlich, dass die Komponenten (Primärelektrode 2, Sekundärelektroden 3, elektrische Leiter 4 und Anschlusselement 5) an einem Trägermaterial 6 angeordnet sind. Das Trägermaterial 6 kann dabei zum Beispiel aus einem Silikon bestehen. Es sind aber auch andere Materialen denkbar, die die anwendungsbedingten Eigenschaften (Biokompatibilität, Flexibilität, elektrisch isolierend, etc.) erfüllen. Es wird deutlich, dass die Primärelektrode 2 und die Sekundärelektroden 3 sowie die elektrischen Leiter 4 in das Trägermaterial 6 eingebettet sind. Weiterhin wird deutlich, dass die elektrischen Leiter 4 der hinteren, vom Anschlusselement 5 weiter entfernten Sekundärelektroden 3 mäanderförmig um die Primärelektrode 1 angeordnet sind, wobei die mäanderförmige Anordnung auf einer gebogenen Bahn um die Primärelektrode 2 verläuft. Auf diese Weise kann die Belastung auf die elektrischen Leiter 4 und damit auch die Ausfallwahrscheinlichkeit der Sekundärelektroden 3 verringert werden. Die elektrischen Leiter 4 sind über eine Verbindungsstelle mit den Sekundärelektroden 3 verbunden, wobei die Verbindungsstelle ein tangentialer Leiteranschluss 7 ist. Der elektrische Leiter 4 kann dabei an die Sekundärelektrode 3 angelötet oder angeschweißt sein, wobei es vorteilhaft ist, wenn die Verbindungsstelle gegen äußere Einflüsse zum Beispiel mit einem Epoxidharz abgedichtet ist.

An der Elektrodenanordnung 1 ist ein Verstärkungselement 8 in Form eines chirurgischen Netzes angeordnet, wobei das Verstärkungselement 8 zwischen den äußeren Schichten des Trägermaterials eingezogen ist. Das Verstärkungselement 8 ist dabei zwischen der Primärelektrode 2 und den Sekundärelektroden 3 zur Verbesserung der Reißfestigkeit angeordnet.

Es ist erkennbar, dass das Anschlusselement 5 an einem Kontaktierungsabschnitt 9 angeordnet ist, wobei der Kontaktierungsabschnitt 9 seitlich von der Elektrodenanordnung 1 abragt und eine größere Länge L als Breite B hat. Die längliche Ausgestaltung des Kontaktierungsabschnittes 9 hat den Vorteil, dass das Anschlusselement 5 von der Primärelektrode 2 und den Sekundärelektroden 3 entkoppelt ist, wobei auf den Kontaktierungsabschnitt 9 einwirkende Kräfte über die Länge L des Kontaktierungsabschnittes 9 soweit kompensiert werden, dass diese Kräfte gar nicht oder schwach an die Elektrodenanordnung 1 weitergeleitet werden. Um die Flexibilität des Kontaktierungsabschnittes 9 zu gewährleisten, kann es von Vorteil sein, wenn das Verstärkungselement 8 nicht am Kontaktierungsabschnitt 9 angeordnet ist und sich lediglich bis zum Übergansgereich zum Kontaktierungsabschnitt 9 hin erstreckt oder sich ähnlich eines Knickschutzes graduell verkleinernd in den Kontaktierungsabschnitt 9 hinein erstreckt.

Die Elektrodenanordnung 1 ist dazu eingerichtet, zum Beispiel zwischen Kopfhaut und Schädel eines Lebewesens implantiert zu werden. Dabei haben die Primärelektrode 2 und die Sekundärelektroden 3 Befestigungsöffnungen 10, wobei in jeweils eine Befestigungsöffnung 10 eine Isolierstoffhülse 11 eingesetzt ist. Die Elektrodenanordnung 1 kann dann über Befestigungselemente wie zum Beispiel über Schrauben durch die Isolierstoffhülse 11 an dem Schädel des Lebewesens befestigt werden. Die Isolierstoffhülse 11 verhindert dabei, dass ein zu großes Drehmoment auf die Primärelektrode 2 oder die Sekundärelektrode 3 übertragen wird und diese damit beschädigt. Des Weiteren wird ein unerwünschter Stromfluss zwischen den Elektroden (Primärelektrode 2 und Sekundärelektroden 3) und dem Befestigungselement vermieden. Die Isolierstoffhülse 11 kann dabei zum Beispiel aus einem Polyetheretherketon bestehen. Durch die Befestigung der Elektrodenanordnung 1 über Befestigungsöffnungen 10 an den jeweiligen Elektroden (Primärelektrode 2 und Sekundärelektroden 3) kann eine exakte Lage der Elektroden gewährleistet werden, die ein erwünschtes elektrisches Feld in der Zielregion erzeugen können. Weiterhin ist erkennbar, dass an dem Kontaktierungsabschnitt 9 zwei Befestigungselemente 12 zur Schraubbefestigung an einem Lebewesen angeordnet sind. Der Kontaktierungsabschnitt 9 kann durch die Befestigungselemente 12 an dem Schädel des Lebewesens befestigt werden.

Deutlich wird, dass die Elektrodenanordnung 1 jeweils zwischen den Sekundärelektroden 3 eine Aussparung 13 hat. Die Aussparungen 13 erhöhen die Flexibilität der Elektrodenanordnung 1, insbesondere im Bereich der Sekundärelektroden 3. Da der Knochen eines Lebewesens in der Regel nicht eben ausgebildet ist, die Elektroden (Primärelektrode 2 und Sekundärelektroden 3) aber exakt am Knochen positioniert werden müssen, ist es vorteilhaft, wenn die Flexibilität der Elektrodenanordnung 1 entsprechend hoch ist, damit die Elektroden 2, 3 in entsprechender Nähe zum Knochen befestigt werden können und die Elektrodenanordnung 1 sich gut der Knochenform anpassen kann.

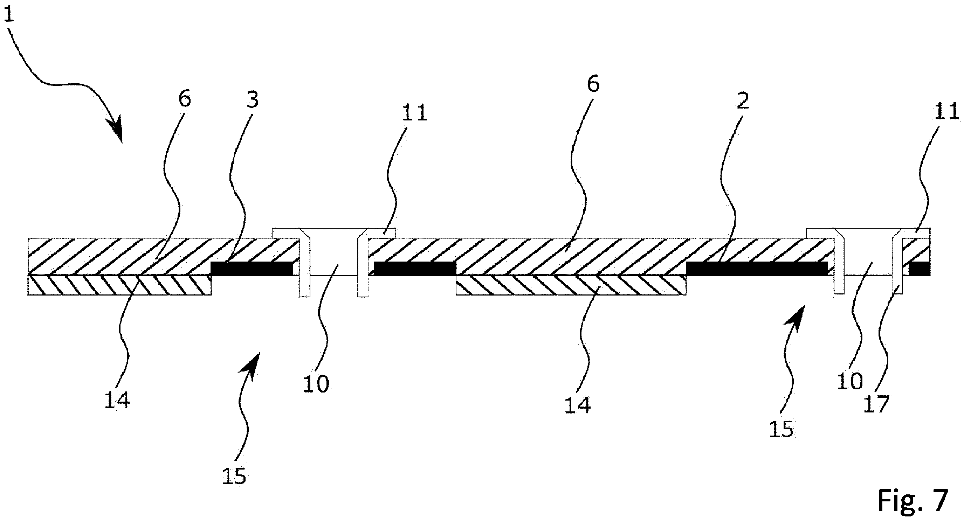

Figur 7 zeigt eine schematische Darstellung einer Primärelektrode 2 und einer Sekundärelektrode 3 in dem Trägermaterial 6 in einer Seitenansicht. Zu erkennen ist, dass die Primärelektrode 2 und die Sekundärelektrode 3 jeweils eine durchgehende Befestigungsöffnung 10 haben, wobei in der Befestigungsöffnung 10 eine Isolierstoffhülse 11 angeordnet ist und wobei die Elektrodenanordnung 1 mittels Befestigungselementen über die Isolierstoffhülse 11 an einer Knochen- oder Gewebestruktur eines Lebewesens befestigt werden kann.

Es wird deutlich, dass das Trägermaterial 6 eine erste umlaufende Dichtlippe 14 an der freiliegenden Kontaktierungsseite 15 der Sekundärelektroden 3 hat. Die umlaufende Dichtlippe 14 kann dabei einstückig aus demselben Material des Trägermaterials 6 gebildet sein. Auf diese Weise kann die Elektrodenanordnung 1 trotz Unebenheiten an der zu implantierenden Stelle befestigt werden, ohne dass es zu Deformationen an den Sekundärelektroden 3 kommt. Dabei ist es auch denkbar, dass die umlaufende Dichtlippe 14 die Sekundärelektrode 3 teilweise überragt, sodass eine verbesserte Fixierung der Sekundärelektrode 3 in dem Trägermaterial 6 ermöglicht wird. Weiterhin erkennbar ist, dass die Sekundärelektroden 3 auf der Kontaktierungsseite 15 freiliegend und auf der anderen Seite vom Trägermaterial 6 isolierend überdeckt sind. Die Sekundärelektroden 3 sind damit im Trägermaterial 6 eingebettet. Dies ermöglicht eine effiziente Isolierung der Sekundärelektroden 3 nach außen und gewährleistet zugleich die Generierung eines erwünschten elektrischen Feldes in einer Körperregion zur Kontaktierungsseite 15 hin. Es ist auch denkbar, dass die Isolierstoffhülse 11 eine zweite umlaufende Dichtlippe 17 hat, die diesen Effekt noch zusätzlich begünstigt. Es ist vorteilhaft, wenn alle Elektroden der Elektrodenanordnung 1 diese Ausgestaltungen aufweisen.

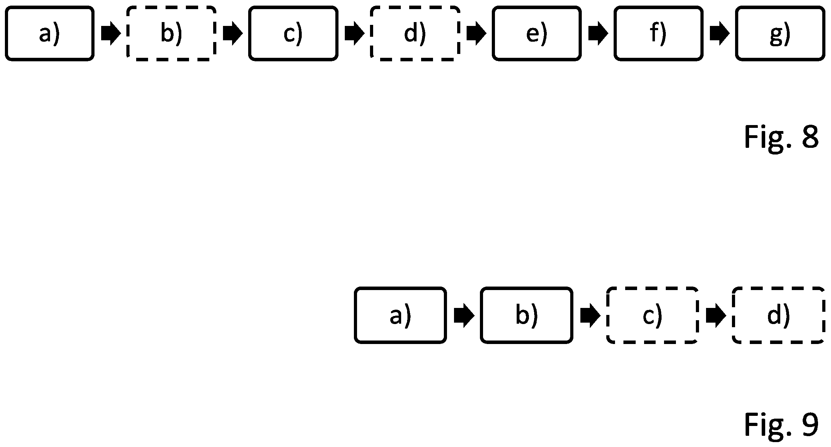

Figur 8 zeigt schematisch ein Verfahren zur Herstellung einer Elektrodenanordnung zur elektrischen Stimulation von Gewebe eines Lebewesens, insbesondere zur Herstellung einer Elektrodenanordnung 1 gemäß den vorbeschriebenen Merkmalen.

In einem Verfahrensschritt a) werden Elektrodenkörper 20 aus einer Folie eines Elektrodenmaterials mit geeigneter Dicke hergestellt, z.B. durch Ausstanzen oder durch Ausschneiden, z.B. mit einem Laser. In einem optionalen Verfahrensschritt b) wird eine Oberflächenbeschichtung und/oder eine Strukturierung der Oberfläche auf zumindest einen Teil der Elektrodenfläche aufgebracht. In einem Verfahrensschritt c) wird an jedem Elektrodenkörper 20 ein elektrischer Leiter 4 angebracht. In einem optionalen Verfahrensschritt d) wird ein Epoxidharz oder ein anderer elektrischer Isolator auf die Verbindungsstelle aufgebracht. In einem Verfahrensschritt e) wird eine Form der Elektrodenanordnung 1 aus dem Trägermaterial 6 gegossen. In einem Verfahrensschritt f) wird der Elektrodenkörper 20 sowie optional der elektrische Leiter 40 und optional das Verstärkungselement 8 eingebettet. In einem Verfahrensschritt g) wird ein elektrischer Leiter 4 am Kontaktierungsabschnitt 9 der Elektrodenanordnung 1 angeschlossen.

Figur 9 zeigt schematisch ein weiteres Verfahren zur Herstellung einer Elektrodenanordnung 1 zur elektrischen Stimulation von Gewebe eines Lebewesens, insbesondere zur Herstellung einer Elektrodenanordnung 1 gemäß den vorbeschriebenen Merkmalen. In einem Verfahrensschritt a) wird ein Trägermaterial 6 bereitgestellt. In einem Verfahrensschritt b) werden ein oder werden mehrere Elektrodenkörper 20 auf dem Trägermaterial 6 durch einen Gasphasenabscheideprozess und/oder durch galvanische Abscheidung erzeugt. In einem optionalen Verfahrensschritt c) wird eine Oberflächenbeschichtung und/oder Strukturierung einer Oberfläche auf zumindest einen Teil der Elektrodenfläche aufgebracht. In einem optionalen Verfahrensschritt d) wird das Trägermaterial 6 in ein weiteres isolierendes Trägermaterial eingebettet.

Die Figuren verstehen sich als ein mögliches Ausführungsbeispiel. Andere Formen der erfindungsgemäßen Lehre sind weiterhin denkbar. Des Weiteren sind die Ausgestaltungen des Ausführungsbeispiels nicht untrennbar miteinander verknüpft, sodass z.B. die Ausführung der Erfindung nicht abhängig von den speziell beschriebenen Ausgestaltungen des Ausführungsbeispiels ist. So ist eine Variabilität zum Beispiel von der Anzahl oder Lagerung der einzelnen Elemente wie zum Beispiel der Primärelektrode 2, Sekundärelektroden 3, Aussparungen 13 und/oder Ausnehmungen 16 jederzeit denkbar.

## Patentansprüche

1. Elektrodenkörper (20) einer Elektrodenanordnung zur elektrischen Stimulation von Gewebe eines Lebewesens, insbesondere für die Neurostimulation, wobei der Elektrodenkörper (20) dazu eingerichtet ist, an einer Stelle zwischen dem Schädel und der Kopfschwarte eines Lebewesens angeordnet zu werden, und wobei der Elektrodenkörper (20) eine Stimulationsoberfläche aufweist, die dazu eingerichtet ist, in Kontakt mit dem Gewebe des Lebewesens gebracht zu werden, um eine elektrische Stimulation des Gewebes durch Wechselstrom- und/oder Gleichstrom-Pulse zu erzeugen, **dadurch gekennzeichnet, dass** der Elektrodenkörper (20) über den Umfang verteilt angeordnete Ausnehmungen (16) zur Fixierung des Elektrodenkörpers (20) in einem Trägermaterial (6) hat.

2. Elektrodenkörper (20) nach einem der vorhergehenden Ansprüche, **dadurch gekennzeichnet, dass** der Elektrodenkörper (20) einen Leiteranschluss (7) hat, über den der Elektrodenkörper (20) durch Löten, Kleben, Schweißen oder eine anderweitige Verbindungstechnik mit einem elektrischen Leiter (4) zur elektrischen Verbindung des Elektrodenkörpers (20) mit einem elektrischen Gerät oder einem anderen Elektrodenkörper (20) verbunden werden kann.

3. Elektrodenkörper (20) nach Anspruch 2, **dadurch gekennzeichnet, dass** der Leiteranschluss (7) als ein tangentialer oder rechtwinkliger Leiteranschluss (7) ausgebildet ist.

4. Elektrodenkörper (20) nach Anspruch 2 oder 3, **dadurch gekennzeichnet, dass** am Leiteranschluss (7) ein elektrischer Leiter (4) zur elektrischen Verbindung des Elektrodenkörpers (20) mit einem elektrischen Gerät oder einem anderen Elektrodenkörper (20) angeschlossen ist, wobei der elektrische Leiter (4) mäanderförmig verläuft.

5. Elektrodenkörper (20) nach einem der vorhergehenden Ansprüche, **dadurch gekennzeichnet, dass** der Elektrodenkörper (20) eine Fixierungsstruktur für die Fixierung des Elektrodenkörpers (20) am Gewebe eines Lebewesens hat, wobei die Fixierungsstruktur eine Isolierstoffhülse (11) aufweist, die zur Isolierung eines mechanischen Befestigungselements gegenüber dem Elektrodenkörper (20) eingerichtet ist.

6. Elektrodenkörper (20) nach Anspruch 5, **dadurch gekennzeichnet, dass** die Fixierungsstruktur wenigstens eine Befestigungsöffnung (10) in dem Elektrodenkörper (20) aufweist, durch die der Elektrodenkörper (20) durch das mechanische Befestigungselement, z.B. eine Schraube, einen Nagel, eine Klammer, einen Faden oder ein ähnliches mechanisches Befestigungselement, am Gewebe des Lebewesens befestigbar ist.

7. Elektrodenkörper (20) nach Anspruch 6, **dadurch gekennzeichnet, dass** die Isolierstoffhülse (11) in der Befestigungsöffnung (10) angeordnet ist.

8. Elektrodenkörper (20) nach einem der Ansprüche 5 bis 7, **dadurch gekennzeichnet, dass** die Isolierstoffhülse (11) eine zweite zumindest teilweise umlaufende Dichtlippe (17) hat.

9. Elektrodenanordnung (1) zur elektrischen Stimulation von Gewebe eines Lebewesens, insbesondere Elektrodenanordnung (1) für die Neurostimulation, mit wenigstens einer Primärelektrode (2) und wenigstens einer Sekundärelektrode (3), wobei eine, mehrere oder alle Primärelektroden (2) durch jeweils einen Elektrodenkörper (20) gebildet sind und/oder eine, mehrere oder alle Sekundärelektroden (3) jeweils durch einen Elektrodenkörper (20) gebildet sind, wobei die Primärelektrode (2) und die Sekundärelektrode (3) an einem gemeinsamen Trägermaterial (6) angeordnet sind, **dadurch gekennzeichnet, dass** die Primärelektrode (2) und/oder zumindest eine der Sekundärelektroden (3) über den Umfang verteilt angeordnete Ausnehmungen (16) zur Fixierung im Trägermaterial (6) hat.

## Claims

1. Electrode body (20) of an electrode arrangement for the electrical stimulation of tissue of a living being, in particular for neurostimulation, wherein the electrode body (20) is configured to be arranged at a location between the skull and the head ridge of a living being, and wherein the electrode body (20) has a stimulation surface which is adapted to be brought into contact with the tissue of the living being in order to generate electrical stimulation of the tissue by alternating current and/or direct current pulses, **characterized in that** the electrode body (20) has recesses (16) arranged distributed over the circumference for fixing the electrode body (20) in a carrier material (6).

2. Electrode body (20) according to one of the preceding claims, **characterized in that** the electrode body (20) has a conductor connection (7) via which the electrode body (20) can be connected by soldering, gluing, welding or another connection technique to an electrical conductor (4) for electrical connection of the electrode body (20) to an electrical device or another electrode body (20).

3. Electrode body (20) according to claim 2, **characterized in that** the conductor connection (7) is configured as a tangential or right-angled conductor connection (7).

4. Electrode body (20) according to claim 2 or 3, **characterized in that** an electrical conductor (4) for the electrical connection of the electrode body (20) to an electrical device or another electrode body (20) is connected to the conductor connection (7), the electrical conductor (4) running in a meandering manner.

5. Electrode body (20) according to one of the preceding claims, **characterized in that** the electrode body (20) has a fixing structure for fixing the electrode body (20) to the tissue of a living being, wherein the fixing structure comprises an insulating material sleeve (11) which is configured to insulate a mechanical fastening element from the electrode body (20).

6. Electrode body (20) according to claim 5, **characterized in that** the fixing structure has at least one fixing opening (10) in the electrode body (20), through which the electrode body (20) can be fixed to the tissue of the living being by the mechanical fixing element, e.g. a screw, a nail, a staple, a thread or a similar mechanical fixing element.

7. Electrode body (20) according to claim 6, **characterized in that** the insulating material sleeve (11) is arranged in the fixing opening (10).

8. Electrode body (20) according to one of claims 5 to 7, **characterized in that** the insulating material sleeve (11) has a second at least partially circumferential sealing lip (17).

9. Electrode arrangement (1) for the electrical stimulation of tissue of a living being, in particular electrode arrangement (1) for neurostimulation, having at least one primary electrode (2) and at least one secondary electrode (3), wherein one, several or all primary electrodes (2) are each formed by an electrode body (20) and/or one, several or all secondary electrodes (3) are each formed by an electrode body (20), wherein the primary electrode (2) and the secondary electrode (3) are attached to a common carrier material (6), several or all secondary electrodes (3) are each formed by an electrode body (20), the primary electrode (2) and the secondary electrode (3) being arranged on a common carrier material (6), **characterized in that** the primary electrode (2) and/or at least one of the secondary electrodes (3) has recesses (16) arranged distributed over the circumference for fixing in the carrier material (6).

## Revendications

1. Corps d'électrode (20) d'un ensemble d'électrodes pour la stimulation électrique du tissu d'un être vivant, en particulier pour la neurostimulation, le corps d'électrode (20) étant conçu pour être placé à un endroit entre le crâne et le cuir chevelu d'un être vivant, et le corps d'électrode (20) présentant une surface de stimulation qui est conçue pour être mise en contact avec le tissu de l'être vivant afin d'engendrer une stimulation électrique du tissu par des impulsions de courant alternatif et/ou de courant continu,
**caractérisé en ce que** le corps d'électrode (20) présente des évidements (16) répartis sur la circonférence, destinés à fixer le corps d'électrode (20) dans un matériau de support (6).

2. Corps d'électrode (10) selon la revendication précédente,
**caractérisé en ce que** le corps d'électrode (20) comprend une borne de conducteur (7) permettant de connecter le corps d'électrode (20) par brasage, collage, soudage ou par une autre technique de connexion à un conducteur électrique (4) pour la connexion électrique du corps d'électrode (20) à un appareil électrique ou à un autre corps d'électrode (20).

3. Corps d'électrode (10) selon la revendication 2,
**caractérisé en ce que** la borne de conducteur (7) est réalisée sous forme de borne de conducteur (7) tangentielle ou à angle droit.

4. Corps d'électrode (20) selon la revendication 2 ou 3,
**caractérisé en ce qu'**un conducteur électrique (4) est connecté à la borne de conducteur (7) pour la connexion électrique du corps d'électrode (20) à un appareil électrique ou à un autre corps d'électrode (20), le conducteur électrique (4) s'étendant en forme de méandres.

5. Corps d'électrode (20) selon l'une des revendications précédentes,
**caractérisé en ce que** le corps d'électrode (20) comprend une structure de fixation destinée à fixer le corps d'électrode (20) au tissu d'un être vivant, la structure de fixation comprenant un manchon en matériau isolant (11) adapté pour isoler un élément de fixation mécanique par rapport au corps d'électrode (20).

6. Corps d'électrode (20) selon la revendication 5,
**caractérisé en ce que** la structure de fixation présente au moins une ouverture de fixation (10) dans le corps d'électrode (20), à travers laquelle le corps d'électrode (20) peut être fixé au tissu de l'être vivant par l'élément de fixation mécanique, par exemple une vis, un clou, une agrafe, un fil ou un élément de fixation mécanique similaire.

7. Corps d'électrode (20) selon la revendication 6,
**caractérisé en ce que** le manchon en matériau isolant (11) est disposé dans l'ouverture de fixation (10).

8. Corps d'électrode (20) selon l'une des revendications 5 à 7,
**caractérisé en ce que** le manchon en matériau isolant (11) comporte une deuxième lèvre d'étanchéité (17) au moins partiellement circonférentielle.

9. Ensemble d'électrodes (1) pour la stimulation électrique du tissu d'un être vivant, en particulier ensemble d'électrodes (1) pour la neurostimulation, comprenant au moins une électrode primaire (2) et au moins une électrode secondaire (3), une, plusieurs ou toutes les électrodes primaires (2) étant formées chacune par un corps d'électrode (20), et/ou une, plusieurs ou toutes les électrodes secondaires (3) étant formées chacune par un corps d'électrode (20), l'électrode primaire (2) et l'électrode secondaire (3) étant disposées sur un matériau de support commun (6),
**caractérisé en ce que**
l'électrode primaire (2) et/ou l'une au moins des électrodes secondaires (3) présentent des évidements (16) répartis sur la circonférence pour la fixation dans le matériau de support (6).
